# EUROPEAN PATENT APPLICATION

(11) **EP 4 364 744 A1**
(43) Date of publication of application: **08.05.2024**
(21) Application number: 22832959.5
(22) Date of filing: 22.06.2022
(51) Int. Cl.: A61K 31/7048, A61P 31/14, C07K 14/165, C12N 15/50

(54) **INHIBITORY AGENT FOR CELL INVASION BY VIRUS**

(30) Priority: 02.07.2021 JP 2021110770
(71) Applicant: Toyo Sugar Refining Co., Ltd., Tokyo 103-0016 (JP)
(72) Inventor: NAKANISHI Akihito, Ichihara-shi, Chiba 290-0046 (JP); TANDIA Mahamadou, Noda-shi, Chiba 278-0027 (JP)
(74) Representative: Dehns
(86) International application number: PCT/JP2022/024859
(87) International publication number: WO 2023/276811

(57) **Abstract**

An object of the present invention is to provide an inhibitor of invasion of virus to cells that inhibits virus invasion into cells. The present invention includes an inhibitor of invasion of virus to cells including at least one component (A) selected from α-glucosylrutin, α-glucosylhesperidin, and α-glucosylnaringin, wherein the virus has a spike protein that binds to Angiotensin-Converting Enzyme 2 (ACE2)

## Description

### Technical Field

The present invention relates to an inhibitor of invasion of virus to cells.

### Background Art

Coronaviruses are viruses that have long been spread in human society as viruses causative of colds. However, in recent years, coronaviruses causing a pandemic worldwide have attracted attention, such as SARS-CoV causing SARS (Severe Acute Respiratory Syndrome) and SARS-CoV-2 causing COVID-19.

Coronaviruses have been classified by the International Committee on Taxonomy of Viruses (ICTV) in the family Coronaviridae of the suborder Cornidovirales of the order Nidovirales. The family Coronaviridae is further divided into the subfamilies Retroviridae and Orthocoronaviridae, and the Orthocoronaviridae includes four genera: α-coronavirus, β-coronavirus, γ-coronavirus, and δ-coronavirus.

Coronaviruses usually have a spike protein (also referred to as S protein), a nucleocapsid protein (also referred to as N protein), a membrane protein (also referred to as M protein), an envelope, an envelope protein (also referred to as E protein), and RNA as components.

In order for the coronavirus to infect a host cell, the spike protein needs to bind to and adsorb to a receptor on the host cell surface, and the genomic RNA needs to invade the cell. At this time, the receptor on the host cell to which the spike protein of the coronavirus binds varies depending on the type of virus. For example, HCoV-NL 63 that is one of cold viruses classified as the α-coronavirus, and SARS-CoV and SARS-CoV-2 classified as the β-coronavirus bind to Angiotensin-Converting Enzyme 2 (ACE2) that is a receptor of a host cell.

In addition, after the spike protein binds to ACE2, in order for the virus to invade a host cell, the virus needs to achieve membrane fusion with the host cell. At that time, it is considered to be important that the spike protein is cleaved by TMPRSS2 (Transmembrane protease serine 2) that is a proteolytic enzyme. Accordingly, it is considered that by inhibiting the activity of TMPRSS2, membrane fusion can be inhibited, and virus invasion into a host cell can be inhibited. Examples of an agent that inhibits the activity of TMPRSS2 and efficiently inhibits virus invasion include Nafamostat and Camostat.

On the other hand, as an antiviral agent using a component derived from a natural product, Patent Literature 1 discloses an antiviral agent containing, as an active ingredient, a cold water extract of hop used for brewing an effervescent alcoholic beverage such as beer. In Patent Literature 1, it is presumed that the antiviral agent contains a predetermined flavonoid glycoside, which adsorbs to the hemagglutinin protein of influenza virus and inhibits binding of the influenza virus to a cell surface receptor, thereby inhibiting invasion of the influenza virus into cells.

Non Patent Literature 1 discloses that from a virtual screening that is one of target evaluations of pharmaceuticals using a computer, Hesperidin may bind to the receptor binding domain (RBD) of the spike protein of SARS-CoV-2.

In addition, Non Patent Literature 2 discloses that from affinity evaluation between a component derived from a natural product such as a flavonoid glycoside and ACE2 by virtual screening using Nafamostat and Captopril that is one of angiotensin converting enzyme inhibitors as control reagents, Resveratrol, Quercetin, Luteolin, Naringenin and the like exhibit high affinity with ACE2.

However, in both of Non Patent Literatures 1 and 2, the effects of these components derived from natural products on SARS-CoV-2 have not been confirmed even in vitro, and in fact the effects have not been verified.

### Citation List

### Patent Literature

Patent Literature 1: WO 2007/099915 A

### Non Patent Literature

Non Patent Literature 1: Canrong Wu et al. Acta Pharmaceutica Sinica B 2020; 10(5): 766-768
Non Patent Literature 2: Vimal K Maurya et al. Virusdisease. 2020 Jun; 31(2): 179-193

### Summary of Invention

### Technical Problem

An object is to provide an inhibitor of invasion of virus to cells that inhibits virus invasion into cells.

### Solution to Problem

The present inventors have intensively studied to solve the above problems. As a result, the present inventors have found that an inhibitor of invasion of virus to cells having the following configuration can solve the above problems, and have completed the present invention.

That is, the present invention includes the following [1] to [6].

[1] An inhibitor of invasion of virus to cells including at least one component (A) selected from α-glucosylrutin, α-glucosylhesperidin, and α-glucosylnaringin, wherein the virus has a spike protein that binds to Angiotensin-Converting Enzyme 2 (ACE2).
[2] The inhibitor of invasion of virus to cells according to [1], wherein the virus is any one selected from SARS-CoV, SARS-CoV-2, and HCoV-NL63.
[3] The inhibitor of invasion of virus to cells according to [1] or [2], wherein the virus is SARS-CoV-2.
[4] The inhibitor of invasion of virus to cells according to any one of [1] to [3], wherein the component (A) contains at least one selected from α-monoglucosylrutin, α-monoglucosylhesperidin, and α-monoglucosylnaringin.
[5] The inhibitor of invasion of virus to cells according to any one of [1] to [4], wherein the content of the component (A) is 50 mass% or more.
[6] The inhibitor of invasion of virus to cells according to any one of [1] to [5], wherein the content of the component (A) is 65 mass% or more.

### Advantageous Effects of Invention

According to the present invention, it is possible to provide an inhibitor of invasion of virus to cells that inhibits virus invasion into cells.

### Brief Description of Drawings

Fig. 1 is a graph showing the amount of luminescence due to luciferase activity when a pseudo SARS-CoV-2 invades Caco-2 cells.
Fig. 2 is a graph showing the amount of luminescence due to luciferase activity when a pseudo SARS-CoV-2 invades ACE2-expressing A549 cells.

### Description of Embodiments

Hereinafter, a description is made of preferred embodiments for carrying out the present invention. Note that the embodiments described below represent examples of representative embodiments of the present invention, and the scope of the present invention is not narrowly interpreted by these embodiments.

The present invention is an inhibitor of invasion of virus to cells including at least one component (A) selected from α-glucosylrutin, α-glucosylhesperidin, and α-glucosylnaringin, wherein the virus has a spike protein that binds to Angiotensin-Converting Enzyme 2 (ACE2).

### <Component (A)>

The inhibitor of invasion of virus to cells of the present invention includes at least one component (A) selected from α-glucosylrutin, α-glucosylhesperidin and α-glucosylnaringin. The component (A) may contain any one or any two of α-glucosylrutin, α-glucosylhesperidin, and α-glucosylnaringin, or may contain all three of α-glucosylrutin, α-glucosylhesperidin, and α-glucosylnaringin. Among them, those containing α-glucosylrutin or α-glucosylnaringin as the component (A) are preferable because the inhibitory effect on invasion of virus to cells is high even at a relatively low concentration.

From the viewpoint of inhibitory effect on invasion of virus to cells, the component (A) preferably contains at least one selected from α-monoglucosylrutin, α-monoglucosylhesperidin, and α-monoglucosylnaringin.

The content of the component (A) contained in the inhibitor of invasion of virus to cells is not particularly limited. For example, the lower limit of the content of the component (A) contained in the inhibitor of invasion of virus to cells of the present invention is, for example, 30 mass%, 35 mass%, 40 mass%, 45 mass%, 50 mass%, 55 mass%, 60 mass%, 65 mass%, 70 mass%, 75 mass%, or 80 mass%. The upper limit of the content of the component (A) contained in the inhibitor of invasion of virus to cells of the present invention is, for example, 100 mass%, 99 mass%, 98 mass%, 95 mass%, 90 mass%, or 85 mass%. As the range of the content of the component (A) contained in the inhibitor of invasion of virus to cells of the present invention, a range in which the lower limit and the upper limit are arbitrarily combined can be arbitrarily set, and for example, a range of 30 to 100 mass%, 60 to 100 mass%, 60 to 90 mass%, or the like can be set. From the viewpoint of inhibitory effect on invasion of virus to cells, the content of the component (A) contained in the inhibitor of invasion of virus to cells of the present invention is preferably 50 mass% or more, more preferably 65 mass% or more.

### [α-Glucosylrutin]

α-Glucosylrutin (also referred to as α glucosylrutin) is a generic term for compounds in which one or more molecules of glucose are added to the glucose residue in the rutinose residue of rutin by α1 → 4 linkage. The α-glucosylrutin in the present invention may be composed of only one type of the compounds having such a structure, or may be a mixture of two or more types thereof.

α-Glucosylrutin can be represented by the following formula (1). In the formula (1), n is 0 or an integer of 1 or more, for example, an integer of 1 to 19.

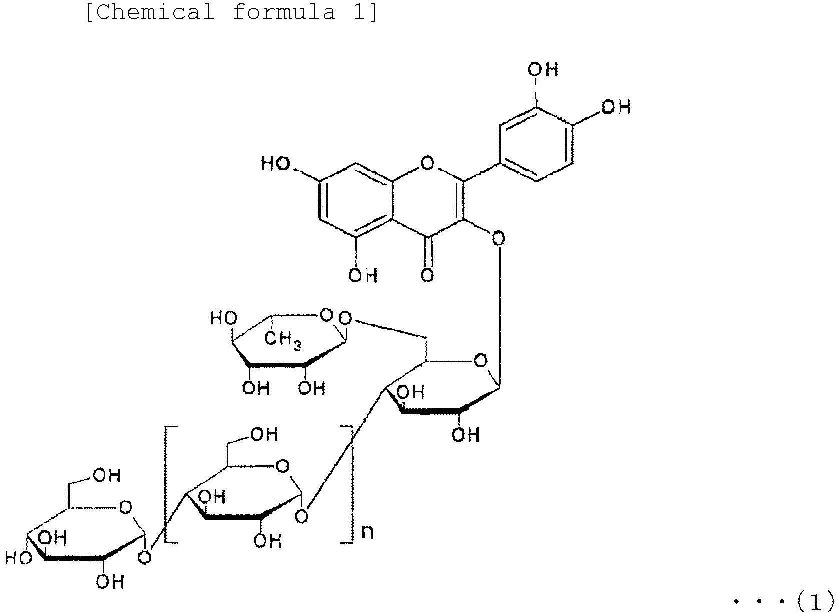

α-Glucosylrutin is a compound contained as a main component in a product known as "enzyme-treated rutin" (sometimes also referred to as "transglycosylated rutin"). Among α-glucosylrutin, one to which only one glucose molecule is bound is referred to as "a-monoglucosylrutin", and one to which two or more glucose molecules are bound is referred to as "α-polyglucosylrutin". That is, in the formula (1), α-monoglucosylrutin is a compound in which n is 0, and α-polyglucosylrutin is a compound in which n is generally 1 to 19.

Enzyme-treated rutin is an aggregate of compounds produced by enzyme treatment for the sugar of rutin, and usually contains a mixture of compounds having different numbers of molecules of glucose bonded to rutin, for example, a mixture composed of α-monoglucosylrutin and α-polyglucosylrutin. In addition, since enzyme-treated rutin is generally produced by enzyme treatment, it may contain unreacted rutin and other derivatives such as isoquercitrin. Note that isoquercitrin is a compound in which β-D-glucose is bonded to the hydroxyl group at the 3 position of the quercetin skeleton, in other words, a compound in which the rhamnose residue in the rutinose residue of rutin is cleaved.

The enzyme-treated rutin is, for example, a product (herein, referred to as "first enzyme-treated rutin") obtained by allowing a glycosyltransferase (an enzyme having the function of adding glucose to rutin, such as cyclodextrin glucanotransferase (CGTase, EC2.4.1.19)) to act on rutin in the coexistence of an α-glucosyl sugar compound (cyclodextrin, partially decomposed starch, etc.) as a sugar donor.

The first enzyme-treated rutin is a composition containing an aggregate composed of various α-glucosylrutin having different numbers of molecules of bound glucose, that is, α-monoglucosylrutin and α-polyglucosylrutin, and rutin that is an unreacted product. If necessary, the first enzyme-treated rutin with the purity of α-glucosylrutin increased (α-glucosylrutin purified product) is obtained by purifying the first enzyme-treated rutin using, for example, a porous synthetic adsorbent and an appropriate eluate to remove the sugar donor and other impurities, and further reduce the content of rutin.

In addition, the first enzyme-treated rutin is treated with an enzyme having a glucoamylase activity that cleaves α-1,4-glucoside bonds for each glucose unit, for example, glucoamylase (EC3.2.1.3), and in α-glucosylrutin to which a plurality of glucose molecules have been added, only one glucose residue directly added to the glucose residue (in the rutinose residue) of rutin itself is left and the other glucose residues are cleaved, whereby enzyme-treated rutin containing a large amount of α-monoglucosylrutin (herein, referred to as "second enzyme-treated rutin") can be obtained. By this enzyme treatment, the glucose residue in the rutinose residue directly bonded to the quercetin skeleton is not cleaved from the quercetin skeleton.

In the inhibitor of invasion of virus to cells of the present invention, in consideration of the effect of the present invention and the like, enzyme-treated rutin that is a composition containing α-glucosylrutin is preferably used, or a composition containing α-glucosylrutin of either the first enzyme-treated rutin or the second enzyme-treated rutin may be used.

In consideration of the effect of the present invention and the like, the enzyme-treated rutin is preferably a mixture containing at least α-glucosylrutin and further containing isoquercitrin. Such a mixture can be produced by the following procedure: (i) the first enzyme-treated rutin described above is prepared, (ii) the first enzyme-treated rutin is treated with an enzyme having glucoamylase activity to convert almost all of α-glucosylrutin into α-monoglucosylrutin, and (iii) the first enzyme-treated rutin is simultaneously treated with an enzyme having rhamnosidase activity to convert almost all of unreacted rutin into isoquercitrin.

Examples of commercially available products of the enzyme-treated rutin include "αG rutin PS", "αG rutin P", and "αG rutin H" which are products of Toyo Sugar Refining Co., Ltd. "αG Rutin PS" is a composition containing 65 mass% α-monoglucosylrutin and 15 mass% isoquercitrin. "αG Rutin P" is a composition containing 60 mass% α-glucosylrutin, 10 mass% rutin, and 1 mass% isoquercitrin.

As the α-glucosylrutin, α-monoglucosylrutin is preferable. This is because since the molecular weight of α-monoglucosylrutin is smaller than the molecular weight of α-polyglucosylrutin, the number of molecules of α-monoglucosylrutin per unit mass is larger, which is considered advantageous in terms of action effect.

The presence of various α-glucosylrutin and other components contained in the enzyme-treated rutin can be confirmed by a chromatogram of HPLC, and the content of each component or the purity of a desired specific component can be calculated from the peak area of the chromatogram.

The method for producing α-glucosylrutin is not particularly limited, and a known method can be used. From the viewpoint of good yield and easy production, the enzyme treatment of rutin as described above is preferable for production. The method for obtaining and preparing rutin is not particularly limited, and a compound generally produced and sold as a reagent or a purified product may be used, or a compound prepared by extraction from raw materials such as citrus (Mikan, orange, etc.) peel or buckwheat seed may be used.

### [α-Glucosylhesperidin]

α-Glucosylhesperidin (also referred to as α glucosylhesperidin) is a generic term for compounds in which one or more molecules of glucose are added to the hydroxyl group in the rutinose unit of hesperidin by an α-1,4 linkage. The α-glucosylhesperidin in the present invention may be composed of only one type of the compounds having such a structure, or may be a mixture of two or more types thereof. Note that hesperidin is a compound in which β-rutinose (6-O-α-L-rhamnosyl-β-D-glucose) is bonded to the hydroxyl group at the 7 position of hesperetin, that is, a hesperetin glycoside.

α-Glucosylhesperidin can be represented by the following formula (2). In the formula (2), n is 0 or an integer of 1 or more, for example, an integer of 1 to 19.

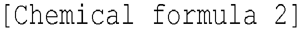

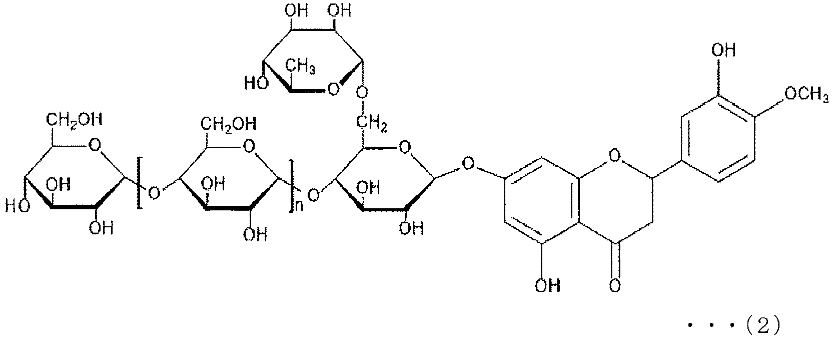

α-Glucosylhesperidin is a compound contained as a main component in a material known as "enzyme-treated hesperidin" (sometimes also referred to as "transglycosylated hesperidin"). Among α-glucosylhesperidin, one to which only one glucose molecule is bound is referred to as "α-monoglucosylhesperidin", and one to which two or more glucose molecules are bound is referred to as "α-polyglucosylhesperidin". That is, in the formula (2), α-monoglucosylhesperidin is a compound in which n is 0, and α-polyglucosylhesperidin is a compound in which n is generally 1 to 19.

Enzyme-treated hesperidin is an aggregate of compounds produced by enzyme treatment for the sugar of hesperidin, and usually contains a mixture of compounds having different numbers of molecules of glucose bonded to hesperidin, for example, a mixture composed of α-monoglucosylhesperidin and α-polyglucosylhesperidin. In addition to α-glucosylhesperidin, unreacted hesperidin or hesperidin derivatives other than α-glucosylhesperidin such as 7-glucosylhesperetin (also referred to as other hesperidin derivatives) may be contained. However, it is preferable that the enzyme-treated hesperidin contain no hesperetin.

Examples of the enzyme treatment for the sugar of hesperidin are as follows.
(1) By allowing a glycosyltransferase to act on hesperidin in the coexistence of a sugar donor such as an α-glucosyl sugar compound (for example, cyclodextrin, partially decomposed starch) to add glucose to a glucose unit of hesperidin by α-1,4 linkage, α-glucosylhesperidin is produced, and a composition containing unreacted hesperidin and α-glucosylhesperidin is obtained (first enzyme-treated hesperidin).
(2) By allowing glucoamylase or the like to act on the α-glucosylhesperidin produced in the above (1) to leave only one glucose molecule while cleaving other glucose molecules from the glucose chain bonded to the glucose unit of hesperidin, α-monoglucosylhesperidin is produced, and a composition containing unreacted hesperidin and α-monoglucosylhesperidin is obtained (second enzyme-treated hesperidin).
(3) By allowing α-L-rhamnosidase to act on the unreacted hesperidin in the above (2) to cleave rhamnose contained in the rutinose unit of hesperidin, 7-glucosylhesperetin is produced, and a composition containing 7-glucosylhesperetin and α-monoglucosylhesperidin is obtained (third enzyme-treated hesperidin).

Examples of the first enzyme treatment include allowing a glycosyltransferase (for example, enzymes having the function of adding glucose to hesperidin, such as cyclodextrin glucanotransferase (CGTase, EC2.4.1.19)) to act on hesperidin in the coexistence of an α-glucosyl sugar compound (for example, cyclodextrin, partially decomposed starch).

In the inhibitor of invasion of virus to cells of the present invention, in consideration of the effect of the present invention and the like, enzyme-treated hesperidin that is a composition containing α-glucosylhesperidin is preferably used, or any composition of first enzyme-treated hesperidin, second enzyme-treated hesperidin, and third enzyme-treated hesperidin may be used.

In consideration of the effect of the present invention and the like, the enzyme-treated hesperidin preferably contains at least α-glucosylhesperidin, and is a mixture further containing one or both of hesperidin and 7-glucosylhesperetin.

Examples of commercially available products of the enzyme-treated hesperidin include "αG hesperidin PS-CC" and "αG hesperidin PA-T" which are products of Toyo Sugar Refining Co., Ltd. "αG Hesperidin PS-CC" contains 80 mass% or more α-monoglucosylhesperidin, and contains 7-glucosyl hesperetin. "αG Hesperidin PA-T" contains 75 mass% or more α-monoglucosylhesperidin, and contains hesperidin.

As the α-glucosylhesperidin, α-monoglucosylhesperidin is preferable. This is because since the molecular weight of α-monoglucosylhesperidin is smaller than the molecular weight of α-polyglucosylhesperidin, the number of molecules of α-monoglucosylhesperidin per unit mass is larger, which is considered advantageous in terms of action effect.

α-Monoglucosylhesperidin can be produced by allowing a sugar hydrolase to act on α-polyglucosylhesperidin to leave only one glucose molecule while cleaving other glucose molecules bound to the hesperidin (second enzyme-treated hesperidin). The sugar hydrolase includes an enzyme having a glucoamylase activity that cleaves an α-1,4-glucoside bond for each glucose unit, for example, glucoamylase (EC3.2.1.3). Note that the ratio of α-monoglucosylhesperidin in α-glucosylhesperidin can be adjusted by the temperature and time conditions of enzyme treatment with glucoamylase. Also, a method for purifying and fractionating α-monoglucosylhesperidin from a mixture of enzyme-treated hesperidin is known.

The presence of various α-glucosylhesperidin, hesperidin, and other components contained in the enzyme-treated hesperidin can be confirmed by a chromatogram of HPLC, and the content of each component or the purity of a desired specific component can be calculated from the peak area of the chromatogram.

The method for producing α-glucosylhesperidin is not particularly limited, and a known method can be used. From the viewpoint of good yield and easy production, the enzyme treatment of hesperidin described above is preferable for production. The method for obtaining and preparing hesperidin is not particularly limited, and a compound generally produced and sold as a reagent or a purified product may be used, or a compound prepared by extraction from raw materials such as citrus (Mikan, orange, etc.) peel may be used.

### [α-Glucosylnaringin]

α-Glucosylnaringin (also referred to as α-glucosylnaringin) is a generic term for compounds in which one or more molecules of glucose are added to the hydroxyl group of naringin. Naringin is a kind of flavonoid having a structure in which neohesperidose (L-rhamnosyl-(α1 → 2)-D-glucose) is β-bonded to the hydroxyl group at the 7 position of the naringenin (5,7,4'-trihydroxyflavanone) skeleton. α-Glucosylnaringin has a structure in which one or more molecules of α-glucose are bonded to at least one of the hydroxyl group at the 3 position (3" position) of the glucose residue in the neohesperidose residue and the hydroxyl group at the 4 position (4' position) of the phenyl group in the naringenin skeleton of the naringin. The α-glucosylnaringin in the present invention may be composed of only one type of the compounds having such a structure, or may be a mixture of two or more types thereof.

α-Glucosylnaringin can be represented by the following formula (3). In the formula (3), m of R¹ and n of R² mean the number of α-glucose residues bonded to the 3" position and the 4' position, respectively, and each independently represent integers of 0 or more and usually 25 or less. However, in order for Formula (3) to represent "α-glucosylnaringin", it is necessary to satisfy m + n ≥ 1, that is, at least one molecule of α-glucose needs to be linked to naringin (when m = n = 0, that is, when both R¹ and R² are -H, Formula (3) represents "naringin").

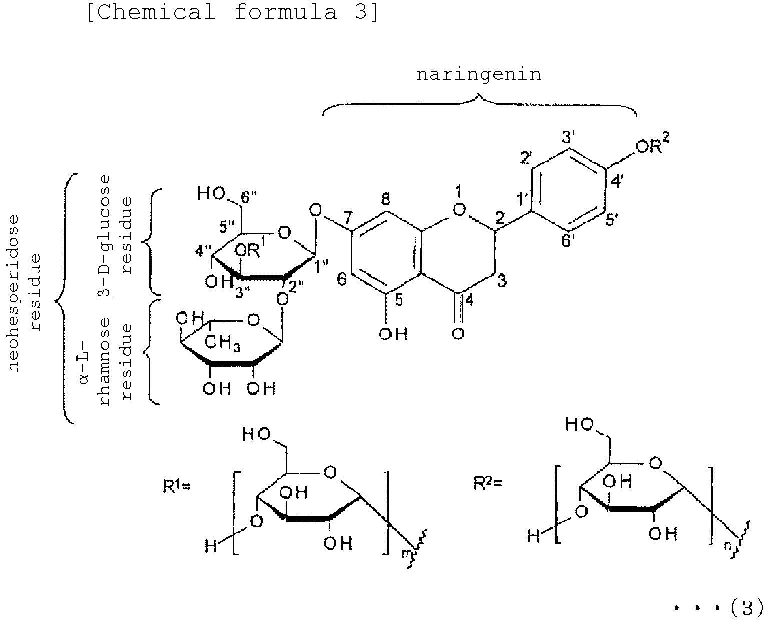

α-Glucosylnaringin is a compound contained as a main component in a material known as "enzyme-treated naringin" (sometimes also referred to as "transglycosylated naringin"). Among α-glucosylnaringin, one to which only one glucose molecule is bound is referred to as "α-monoglucosylnaringin", and one to which two or more glucose molecules are bound is referred to as "α-polyglucosylnaringin".

The enzyme-treated naringin is, for example, a product (herein, referred to as "first enzyme-treated naringin") obtained by reacting a mixture of naringin and a sugar donor (for example, dextrin) with a glycosyltransferase (for example, cyclodextrin glucosyltransferase), which product is an aggregate of various compounds in which one or more molecules of glucose are added to the hydroxyl group of naringin. Accordingly, the enzyme-treated naringin usually includes a mixture of compounds that differ in the number of glucose molecules bound to the naringin, such as a mixture composed of α-monoglucosylnaringin and α-polyglucosylnaringin. In addition to α-glucosylnaringin, unreacted naringin or naringin derivatives other than α-glucosylnaringin such as 7-glucosylnaringenin (also referred to as other naringin derivatives) may be contained.

For a basic method for producing the first enzyme-treated naringin, for example, JP H04-13691 A can be referred to. If necessary, the first enzyme-treated naringin with the purity of α-glucosylnaringin increased (α-glucosylnaringin purified product) is obtained by purifying the first enzyme-treated naringin using, for example, a porous synthetic adsorbent and an appropriate eluate to remove the sugar donor and other impurities, and further reduce the content of naringin.

As the α-glucosylnaringin, at least one α-glucosylnaringin selected from those in which one molecule of α-glucose is linked to the 3" position of naringin and/or one molecule of α-glucose is linked to the 4' position of naringin, that is, 3"-α-monoglucosylnaringin (in the formula (3), m = 1, n = 0), 4'-α-monoglucosylnaringin (m = 0, n = 1), and 3"-4'-α-diglucosylnaringin (m = 1, n = 1) is preferable, and among them, 3"-α-monoglucosylnaringin is more preferable. This is because since the molecular weight of α-monoglucosylnaringin is smaller than the molecular weight of α-polyglucosylnaringin, the number of molecules of α-monoglucosylnaringin per unit mass is larger, which is considered advantageous in terms of action effect.

The enzyme-treated naringin containing a large amount of the three types of α-mono/diglucosylnaringin (herein, referred to as "second enzyme-treated naringin") can be obtained, for example, by treating the above-described first enzyme-treated naringin with an enzyme having glucoamylase activity, and cleaving a sugar chain in which two or more molecules of α-glucose are bonded to each other by an α-1,4 linkage, the sugar chain having been transferred to the 3" position and/or the 4' position of naringin by the glycosyltransferase, while leaving only an α-glucose residue corresponding to one molecule at the base. Furthermore, the second enzyme-treated naringin is treated with an enzyme having α-glucosidase activity, and the α-glucose residue corresponding to one molecule that is directly bonded to the hydroxyl group at the 4' position is cleaved, whereby an enzyme-treated naringin leaving 3"-α-monoglucosylnaringin and containing little or no 4'-α-monoglucosylnaringin and 3"-4'-α-diglucosylnaringin (herein, referred to as "third enzyme-treated naringin") can be obtained. For a basic method for producing the second and third enzyme-treated naringins, for example, JP 2002-199896 A can be referred to.

Furthermore, 7-glucosylnaringenin is produced by allowing α-L-rhamnosidase to act on the third enzyme-treated naringin to cleave rhamnose contained in the rutinose unit of the naringin, whereby an enzyme-treated naringin containing 7-glucosylnaringenin and α-monoglucosylnaringin (herein, referred to as "fourth enzyme-treated naringin") is obtained.

In addition, when the transglucosidase is caused to act on the first enzyme-treated naringin, since the transglucosidase is an enzyme having both glucoamylase activity and α-glucosidase activity, the third enzyme-treated naringin rich in 3"-α-monoglucosylnaringin is obtained by a one-step treatment instead of the two-step treatment as described above. Furthermore, if necessary, a treatment may be performed in which an enzyme having β-glucosidase activity is allowed to act on the third enzyme-treated naringin (which enzyme may be simultaneously allowed to act with the transglucosidase on the first enzyme-treated naringin) to cleave the neohesperidose residue of unreacted naringin (whose sugar chain has not been modified by glycosyltransferase) dissolved in a small amount in an aqueous solution from naringenin that is an aglycone of the neohesperidose residue. Since naringenin produced by such a treatment has lower solubility than that of naringin, naringenin can be easily removed from the aqueous solution through precipitation, so that 3"-α-monoglucosylnaringin can be collected with higher purity from the aqueous solution.

In the inhibitor of invasion of virus to cells of the present invention, in consideration of the effect of the present invention and the like, an enzyme-treated naringin which is a composition containing α-glucosylnaringin is preferably used, or any composition of first enzyme-treated naringin, second enzyme-treated naringin, third enzyme-treated naringin, and fourth enzyme-treated naringin may be used.

In consideration of the effect of the present invention and the like, the enzyme-treated naringin preferably contains at least α-glucosylnaringin, and is a mixture further containing one or both of naringin and 7-glucosylnaringenin.

The presence of various α-glucosylnaringin, naringin, and other components contained in the enzyme-treated naringin can be confirmed by a chromatogram of HPLC, and the content of each component or the purity of a desired specific component can be calculated from the peak area of the chromatogram.

The method for producing α-glucosylnaringin is not particularly limited, and a known method can be used. From the viewpoint of good yield and easy production, the enzyme treatment of naringin described above is preferable for production. The method for obtaining and preparing naringin is not particularly limited, and a compound generally produced and sold as a reagent or a purified product may be used, or a compound prepared by extraction from raw materials such as citrus (Natsumikan, grapefruit, etc.) peel may be used.

### <Virus>

In the present invention, the virus having a spike protein that binds to Angiotensin-Converting Enzyme 2 (hereinafter, referred to as "ACE2") is not particularly limited, but may be any virus as long as it can allow viral genomic DNA or genomic RNA to invade cells through binding between the viral spike protein and ACE2 that is a receptor of a host cell, and examples thereof include SARS-CoV, SARS-CoV-2 or HCoV-NL63, BatCoV-WIV1, BatCoV-WIV16, BatCoV-RS4231, and BatCoV-RsSHC014. Among them, from the viewpoint of the inhibitory effect of the present invention, SARS-CoV, SARS-CoV-2, or HCoV-NL 63 is preferable, and SARS-CoV-2 is more preferable.

The spike protein (also referred to as S protein, spike glycoprotein, etc.) is a glycoprotein that usually penetrates the envelope of the virus surface. The spike protein is not particularly limited as long as it can bind to ACE2, but preferably has a subunit or domain having a receptor binding domain (RBD) that binds to ACE2 and a subunit or domain having a fusion peptide for fusing with a host cell membrane.

The sequence information of the amino acid sequence of the spike protein and base sequence thereof can be obtained from, for example, databases such as GenBank and GISAID (Global Initiative on Sharing All Influenza Data). Examples of the amino acid sequence of the spike protein include SEQ ID NO: 1 (SARS-CoV), SEQ ID NO: 2 (SARS-CoV-2), or SEQ ID NO: 3 (HCoV-NL63).

As long as the spike protein can bind to ACE2, the spike protein may be a mutant spike protein in which 1 to 3, preferably 1 to 2, more preferably 1 amino acid is deleted, substituted, or added in its amino acid sequence.

The structure of the spike protein is not particularly limited as long as the spike protein can bind to ACE2, but for example, when the receptor binding domain has a down structure or an up structure, it is preferable that the receptor binding domain have an up structure from the viewpoint of affinity with ACE2.

A virus having a spike protein that binds to ACE2 also includes a mutant strain in which the spike protein is mutated. Examples of the mutant strain of SARS-CoV-2 include mutant strains of N501Y type, E484K type, L452R type, E484Q type, K417N type, H417T type, N439K type, D614G type, A222V type, Y453F type, P681H type, A570D type, T716I type, S982A type, A1708D type, A701V type, D80A type, L18F type, R246I type, D215G type, delH69V70 type, delY144 type, Q27stop type, and L242_244L type.

The cell that the virus invade is not particularly limited as long as it has ACE2 on the cell surface, but examples thereof include cells such as from brain, heart, aorta, lung, esophagus, stomach, duodenum, jejunum, ileum, cecum, colon, rectum, kidney, skeletal muscle, spleen, thymus gland, trachea, placenta, bladder, uterus, prostate, testis, ovary, pancreas, adrenal gland, thyroid gland, salivary gland, tongue, gingiva, mammary gland, bone marrow, and blood vessel. The organism from which the host cell is derived is not particularly limited, but is preferably derived from human, bat, pangolin, pig, cat, dog, cow, mouse, rat, or chicken, and more preferably derived from human.

### <Inhibitor of invasion of virus to cell>

The inhibitor of invasion of virus to cells only needs to contain at least one component (A) selected from α-glucosylrutin, α-glucosylhesperidin, and α-glucosylnaringin, and may be composed of only the component (A), or may further contain known optional components such as an excipient, a stabilizer, a wetting agent, and an emulsifier as long as the inhibitory effect on invasion of virus to cells by the component (A) is not hindered. The inhibitor of invasion of virus to cells may be at least one selected from enzyme-treated rutin, enzyme-treated hesperidin, and enzyme-treated naringin, which are compositions containing the component (A), or may contain at least one selected from enzyme-treated rutin, enzyme-treated hesperidin, and enzyme-treated naringin.

The inhibitor of invasion of virus to cells may have an action of preventing a virus having a spike protein that binds to ACE2 from binding to and adsorbing to ACE2 of a cell, and genomic DNA or genomic RNA of the virus from invading the cell. The action of inhibiting invasion of virus to cells by an inhibitor of invasion of virus to cells can be confirmed by a change in luminescence value due to luciferase activity of the virus that has invaded a host cell having ACE2 in an in vitro inhibition test of invasion to cells using a pseudovirus, as described in Examples. That is, when the group using the inhibitor of invasion of virus to cells had a smaller luminescence value due to luciferase activity compared to the group not using the inhibitor of invasion of virus to cells, it is indicated that invasion of the virus into a host cell was inhibited by the inhibitor of invasion of virus to cells.

The inhibition rate of invasion of virus to cells is not particularly limited, but in an inhibition test of invasion to cells using a pseudovirus, when the inhibition rate when the inhibitor of invasion of virus to cells of the present invention is not added is 0%, the inhibition rate when the inhibitor of invasion of virus to cells of the present invention is added is preferably 10% or more, more preferably 30% or more, and still more preferably 50% or more. The inhibition rate of invasion of virus to cells can be expressed by the following formula.

Inhibition rate of invasion of virus to cell (%) = 100 × {1 - (luminescence value of group to which inhibitor of invasion of virus to cell has been added)/(luminescence value of group to which inhibitor of invasion of virus to cell has not been added)}

The pseudovirus can be produced by a known method, or a commercially available product can be purchased for use. Examples of the commercially available product include Lenti-X (TM) SARS-CoV-2 (manufactured by Takara Bio Inc.), SARS-CoV pseudovirus, and HCov-NL 63 pseudovirus (manufactured by Vector Builder).

### [Use]

The use of the inhibitor of invasion of virus to cells is not particularly limited, but since the inhibitor of invasion of virus to cells has an action of inhibiting invasion of virus into cells, the inhibitor can be used for the purpose of preventing viral infection, for example, preventing infection with SARS-CoV, SARS-CoV-2, and HCoV-NL63.

The dosage of the inhibitor of invasion of virus to cells may be appropriately selected depending on the type of viral infection, the age, sex, and race of an administered subject, and the like. For example, when the inhibitor of invasion of virus to cells is orally administered, the amount of the component (A) is preferably 10 mg to 1000 mg, more preferably 100 mg to 300 mg per day. The number of times of administration of the inhibitor of invasion of virus to cells may be one or more, and the frequency of administration per day may be, for example, 1 to 3 times per day, or may be 2 or 3 times. The administration period of the inhibitor of invasion of virus to cells may be appropriately selected depending on the type of viral infection, the age, sex, and race of an administered subject, and the like. From the viewpoint of the inhibitory effect on invasion of virus to cells, the administration period is preferably 1 day to 90 days, more preferably 14 days to 60 days.

### [Formulation]

The inhibitor of invasion of virus to cells may be administered to a living body as it is, or may be administered as a formulation in which an effective amount of the inhibitor of invasion of virus to cells is blended together with a pharmaceutically acceptable carrier. Examples of the formulation include foods and beverages, pharmaceuticals, and quasi-pharmaceutical products. The administration method is not particularly limited, and can be oral or parenteral administration. The formulation is preferably orally administered because it is easy to administer.

In the case of oral administration, the formulation may be foods or beverages (including health functional foods such as foods for specified health use, food with nutrient function claims, and foods with functional claims, and other so-called health foods and supplements), pharmaceutical products, quasi-pharmaceutical products, or the like.

Formulations for oral use can be solid or liquid (including pasty). The dosage form is not limited, and specific examples of the solid formulation include a powder, a granule, a tablet, a capsule, and a troche. In addition, examples of the liquid formulation include an internal liquid, a suspension, an emulsion, a syrup, a drink, and the like, and these dosage forms and other dosage forms are appropriately selected depending on the purpose.

In the case of parenteral administration, the formulation can be an injection, a suppository, or the like.

The formulation can be produced by adding the inhibitor of invasion of virus to cells to a formulation according to a method generally used for such a formulation. The inhibitor of invasion of virus to cells may be added at the initial stage of the production process of the formulation, or may be added at the middle or final stage of the production process, and the addition method may be appropriately selected from mixing, kneading, dissolution, immersion, dispersion, spraying, application, and the like depending on the aspect of the formulation.

The component (A), which is an active ingredient of the inhibitor of invasion of virus to cells, has good water solubility, and thus can be uniformly dissolved or dispersed even when added to water or a formulation containing a large amount of water.

The amount of the inhibitor of invasion of virus to cells to be blended into the formulation may be appropriately selected depending on the type of disease or the degree of symptom in which the inhibitory activity of invasion of virus to cells is involved, but from the viewpoint of ease of administration and stability in the formulation, the amount of the component (A) is preferably 5 to 98 mass%, more preferably 20 to 70 mass%, still more preferably 30 to 50 mass%.

### Examples

Hereinafter, a more specific description is made of the present invention based on Examples, but the present invention is not limited to the Examples, and can be appropriately modified and implemented without changing the gist thereof.

### [Preparation example 1]

Pseudo SARS-CoV-2 was prepared as follows, which had SARS-CoV-2 spike proteins on the envelope surface, and exhibited luciferase activity after invasion into the host cell.

### (1) Preparation of lentiviral vector plasmid (pCDH-fLuc) for expression of fLuc (firefly luciferase)

The pCDH-EF1-MCS-(PGK-copGFP-T2A-Puro) plasmid (manufactured by System Biosciences) purified using the PureLink Expi Endotoxin-Free Maxi Plasmid Purification Kit (manufactured by Thermo Fisher Scientific) was cut with EcoRI (EcoRI-HF: manufactured by New England Biolabs) and BamHI (BamHI-HF: manufactured by New England Biolabs) to prepare a linear plasmid vector.

A product obtained by column purification of the linear plasmid vector and a synthesis gene (SEQ ID NO: 4) for fLuc expression were ligated using In-Fusion (registered trademark) HD cloning kit (manufactured by Takara Bio Inc.) according to the instruction attached to the kit, and the obtained vector plasmid for lentivirus preparation was used to transform Escherichia coli.

Colony PCR using various primers (CD 813_F1 (SEQ ID NO: 5), CD 813_R1 (SEQ ID NO: 6)) was performed on E. coli that had become drug resistant, and an amplified product of about 1.8 kbp containing a target gene was confirmed. The amplified product was subjected to sequence analysis using various primers (CD 813_F1, CD 813_R1), which confirmed that pCDH-fLuc had the sequence as designed.

E. coli carrying this vector plasmid for lentivirus preparation was cultured in liquid, and a lentivirus vector plasmid for fLuc expression (pCDH-fLuc) was prepared by Maxi-prep.

### (2) Preparation of plasmid expressing SAR-CoV-2 (pCD5 spCoV2)

The pcDNA 3.1/Hygro plasmid (manufactured by Thermo Fisher Scientific) was cut with NheI (NheI-HF: manufactured by New England Biolabs) and AflII (manufactured by New England Biolabs) to prepare a linear plasmid vector. After column purification of the linear plasmid vector, the purified linear plasmid vector and a synthesis gene (SEQ ID NO: 9) obtained by ligating a signal sequence of CD5 (SEQ ID NO: 7) and an S gene of SARS-CoV-2 (SEQ ID NO: 8) were ligated by In-Fusion HD cloning kit (manufactured by Takara Bio Inc.), and the obtained product was used to transform E. coli.

Colony PCR using plasmid-specific primers (pcDNA3_F1 (SEQ ID NO: 10) and pcDNA3_R2 (SEQ ID NO: 11)) was performed on E. coli that had become drug resistant, and an amplification product of about 4 kbp containing the target gene was confirmed. Sequence analysis was performed on the amplified product using primers of CMV_F4 (SEQ ID NO: 12), SARS-CoV-2_Seq1 (SEQ ID NO: 13), SARS-CoV-2_Seq2 (SEQ ID NO: 14), SARS-CoV-2_Seq3 (SEQ ID NO: 15), and pcDNA3_R2, which confirmed that pCD5spCoV2 was a sequence as designed.

E. coli carrying this plasmid vector was cultured in liquid, and a plasmid (pCD5spCoV2) was prepared by Maxi-prep.

### (3) Preparation of pseudo SARS-CoV-2 (pseudo coronavirus)

Lenti-X 293T cells (manufactured by Takara Bio Inc.) were cultured using Opti-MEM (manufactured by Thermo Fisher Scientific) containing 5% FBS and 1 mM sodium pyrubate as a basic medium. Thereafter, Lenti-X 293T cells (1.8 × 10⁷ cells) were suspended in 34 mL of the basic medium, followed by seeding in a 15 cm dish.

On the next day, transformation was performed using Lipofectamine 3000 Transfection Reagent (manufactured by Thermo Fisher Scientific) as follows (the composition of the following mixture represents a composition for one 15 cm dish, and transformation was performed on three 15 cm dishes).

A mixture was obtained by mixing 3.9 mL of Opti-MEM (manufactured by Thermo Fisher Scientific), 14.4 µg of pPACKH1-GAG (component of pPACKH1 HIV Lentivector Packaging Kit (manufactured by System Biosciences)), 7.2 µg of pPACKH1-REV (component of pPACKH1 HIV Lentivector Packaging Kit (manufactured by System Biosciences)), 11.2 µg of the pCDH-fLuc, 12.2 µg of the pCD5spCoV2, and 91 µL of P3000 reagent (component of Lipofectamine 3000 Transfection Reagent (manufactured by Thermo Fisher Scientific)). To the obtained mixture, a mixture of 3.9 mL of Opti-MEM and 107 µL of Lipofectamine 3000 reagent was added, and the resultant mixture was kept at room temperature for 10 minutes to form a DNA/Lipofectamine 3000 complex.

Subsequently, a solution containing the complex (7.8 mL) was added to the Lenti-X 293T cells. Six hours after addition of the complex, the complex was replaced with 21 mL of Opti-MEM containing 5% FBS and 1 mM sodium pyruvate, and 24 hours after culture, the culture supernatant (1) was collected and stored at 4°C.

The culture medium after collection of the culture supernatant (1) was replaced with 21 mL of Opti-MEM containing 5% FBS and 1 mM sodium pyruvate, followed by 24 hours culturing. After the culture, the culture supernatant (2) was collected.

A mixture of the culture supernatant (1) and the culture supernatant (2) was obtained as a pseudo SARS-CoV-2 solution for use in the following experiment. The pseudo SARS-CoV-2 solution was stored at -80°C until use.

### [Example 1]

### <Inhibition test of invasion of pseudo SARS-CoV-2 to cell using Caco-2 cell>

The inhibitory effect of invasion of the pseudo SARS-CoV-2 to cells was verified when Caco-2 cells (human colon cancer-derived cells) and the pseudo SARS-CoV-2 were cultured in a medium containing αG hesperidin PA-T, αG rutin PS, or αG naringin PS (fourth enzyme-treated naringin).

### [Method]

As samples, αG rutin PS (manufactured by Toyo Sugar Refining Co., Ltd.) was used as α-glucosylrutin (αG rutin), αG hesperidin PA-T (manufactured by Toyo Sugar Refining Co., Ltd.) was used as α-glucosylhesperidin (αG hesperidin), and αG naringin PS (fourth enzyme-treated naringin; 75 mass% α-monoglucosylnaringin, 10 mass% 7-glucosylnaringenin) was used as α-glucosylnaringin (αG naringin).

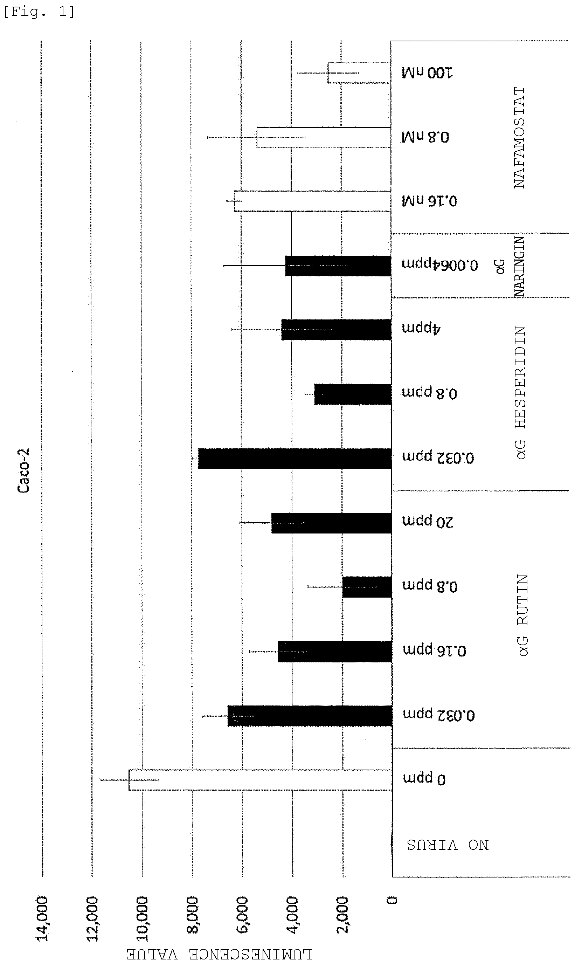

Caco-2 cells (manufactured by ATCC) were prepared in the Eagle's minimal essential medium (composition: MEM non-essential amino acid solution) so as to be 6 × 10³ cells/well, followed by seeding on a 96 well plate and culturing at 37°C in a 5% CO₂ environment. Twenty-four hours after seeding, 10 µL of a sample at each concentration (final concentration in the medium) shown in Fig. 1 was added to each well.

Two hours after addition of the sample, 140 µL of the pseudo SARS-CoV-2 solution was added to each well, while 140 µL of the basic medium was added to a well to which the pseudo SARS-CoV-2 solution was not added, followed by culturing of Caco-2 cells for 46 hours. After the culturing, the medium was replaced, and the pseudo SARS-CoV-2 solution and the sample were removed, followed by further culturing for 24 hours.

After the culturing, the luminescence value due to luciferase activity of the pseudo SARS-CoV-2 that invaded the cell was measured.

### [Results]

The results of the luminescence value are shown in Fig. 1. The inhibition rate of invasion to cells calculated from the luminescence value is shown in Table 1. The inhibition rate of invasion to cells was calculated as follows. Inhibition rate of invasion to cell (%) = 100 × {1 - (luminescence value in presence of sample)/(luminescence value in absence of sample)}. The number of N per experimental condition was 3, the p value was calculated by t-test using the case where the virus was added but the sample was not added (0 ppm) as a control, and p < 0.05 was defined as having a significant difference. From the above results, it has been shown that each sample has an effect of inhibiting pseudo SARS-CoV-2 from invading cells at any concentration.

### [Comparative example 1]

In the same manner as in Example 1 except that Nafamostat (manufactured by Tokyo Chemical Industry Co., Ltd.) was used instead of the above sample, the luminescence value due to luciferase activity was measured to calculate the inhibition rate of invasion to cell (%). The results are shown in Fig. 1 and Table 1.

**[Table 1]**

| Sample | Concentration | Inhibition rate of invasion to cell (%) | P value | Significant difference |
|---|---|---|---|---|
| αG Rutin PS | 0.032 ppm | 37.6 | 0.023 | Detected |
| | 0.16 ppm | 56.6 | 0.007 | Detected |
| | 0.80 ppm | 81.2 | 0.003 | Detected |
| | 20 ppm | 54.3 | 0.010 | Detected |
| αG Hesperidin PA-T | 0.032 ppm | 26.8 | 0.031 | Detected |
| | 0.80 ppm | 70.9 | 0.001 | Detected |
| | 4.0 ppm | 58.4 | 0.020 | Detected |
| αG Naringin PS | 0.0064 ppm | 59.9 | 0.032 | Detected |
| Nafamostat | 0.16 nM | 40.4 | 0.008 | Detected |
| | 0.80 nM | 48.9 | 0.034 | Detected |
| | 100 nM | 76.1 | 0.003 | Detected |

### [Example 2]

### <Inhibition test of invasion of pseudo SARS-CoV-2 to cell using ACE2-expressing A549 cell>

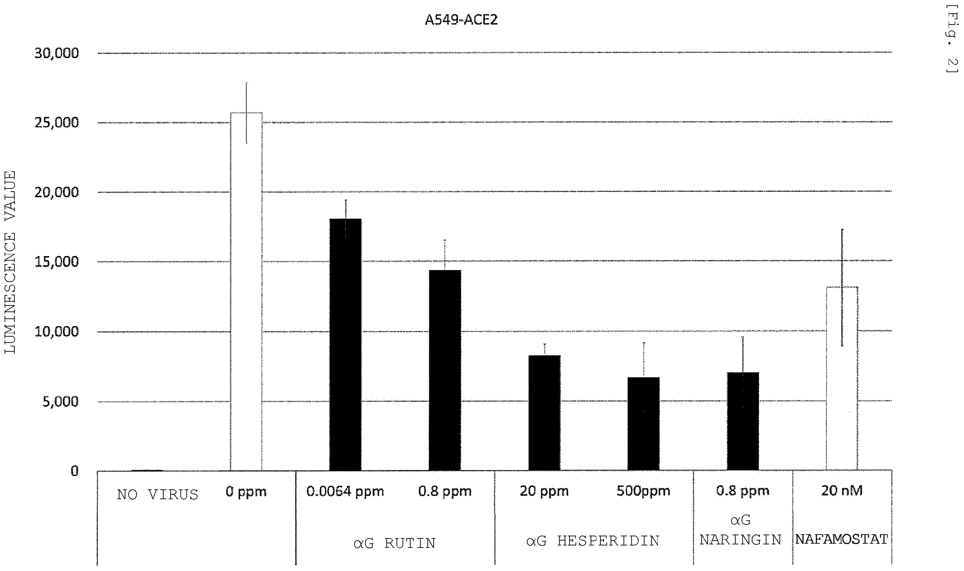

In the same manner as in Example 1 except that ACE2-expressing A549 cells modified to forcibly express ACE2 by the method described later were used instead of Caco-2 cells, the luminescence value due to luciferase activity was measured to calculate the inhibition rate of invasion to cell (%). The results are shown in Fig. 2 and Table 2. From the above results, it has been shown that each sample has an effect of inhibiting pseudo SARS-CoV-2 from invading cells at any concentration.

**[Table 2]**

| Sample | Concentration | Inhibition rate of invasion to cell (%) | P value | Significant difference |
|---|---|---|---|---|
| αG Rutin PS | 0.0064 ppm | 29.6 | 0.013 | Detected |
| | 0.80 ppm | 44.1 | 0.007 | Detected |
| αG Hesperidin PA-T | 20 ppm | 67.9 | 0.005 | Detected |
| | 500 ppm | 73.9 | 0.001 | Detected |
| αG Naringin PS | 0.80 ppm | 72.7 | 0.001 | Detected |
| Nafamostat | 20 nM | 49.0 | 0.019 | Detected |

### [Preparation example 2]

ACE2-expressing A549 cells (A549-ACE2) were prepared as follows.

### (1) Preparation of ACE2 plasmid vector

The pcDNA3.1/Hygro plasmid (manufactured by Thermo Fisher Scientific) was cut with NheI (NheI-HF: manufactured by New England Biolabs) and AflII (manufactured by New England Biolabs), followed by column purification. Then, each purified product and a synthetic gene (SEQ ID NO: 16) for generation of ACE2 were ligated using In-Fusion HD cloning kit (manufactured by Takara Bio Inc.) according to the instruction attached to the kit, and the obtained product was used to transform E. coli.

Colony PCR was performed on E. coli that had become drug resistant, and an amplification product of about 2.8 kbp containing the target gene was confirmed. Sequence analysis was performed on the amplified product using primers of CMV_F4 (SEQ ID NO: 12), Hs_ACE2_Seq1 (SEQ ID NO: 17), and pcDNA3_R2 (SEQ ID NO: 11), which confirmed that the sequence was as designed (pcDNA 3.1-Hyg-ACE2). E. coli carrying the plasmid vector was cultured in liquid, and pcDNA 3.1 Hyg-ACE2 plasmid was prepared by Maxi-prep to yield a DNA for transfection.

### (2) Preparation of ACE2-expressing A549 cell

The pcDNA 3.1 Hyg-ACE2 plasmid was transfected into A549 cells (manufactured by JCRB Cell Bank), followed by selection in a growth medium containing 50 mg/mL Hygromycin B (manufactured by FUJIFILM Wako Pure Chemical Corporation) to yield cells into which the pcDNA 3.1-Hyg-ACE2 plasmid had been stably introduced (A549-ACE2). Thereafter, 32 clones were obtained by limiting dilution method, and then from the clone cells and the negative control (pcDNA 3.1-Hyg-ACE2 plasmid-unintroduced A549), RNA was extracted and purified, followed by cDNA synthesis. For the A549-ACE2 clone, primers of Hs_ACE2_RTF1 (SEQ ID NO: 18) and Hs_ACE2_RTR1 (SEQ ID NO: 19) were used, and the expression level of mRNA of ACE2 was quantitatively analyzed by real-time PCR to confirm expression of ACE2.

## Claims

1. An inhibitor of invasion of virus to cells comprising at least one component (A) selected from α-glucosylrutin, α-glucosylhesperidin, and α-glucosylnaringin, wherein the virus has a spike protein that binds to Angiotensin-Converting Enzyme 2 (ACE2).

2. The inhibitor of invasion of virus to cells according to claim 1, wherein the virus is any one selected from SARS-CoV, SARS-CoV-2, and HCoV-NL63.

3. The inhibitor of invasion of virus to cells according to claim 1, wherein the virus is SARS-CoV-2.

4. The inhibitor of invasion of virus to cells according to claim 1, wherein the component (A) contains at least one selected from α-monoglucosylrutin, α-monoglucosylhesperidin, and α-monoglucosylnaringin.

5. The inhibitor of invasion of virus to cells according to claim 1, wherein the content of the component (A) is 50 mass% or more.

6. The inhibitor of invasion of virus to cells according to claim 1, wherein the content of the component (A) is 65 mass% or more.
